# EUROPEAN PATENT SPECIFICATION

(11) **EP 4 274 475 B1**
(45) Date of publication and mention of the grant of the patent: **16.10.2024**
(21) Application number: 22700462.9
(22) Date of filing: 04.01.2022
(51) Int. Cl.: A61B 5/0507, A61B 5/11, A61B 5/00, G01S 13/56

(54) **DETECTION SYSTEM AND METHOD FOR DETECTING MOTION OF A SUBJECT**
DETEKTIONSSYSTEM UND VERFAHREN ZUR DETEKTION DER BEWEGUNG EINES SUBJEKTS
SYSTÈME DE DÉTECTION ET PROCÉDÉ DE DÉTECTION DE MOUVEMENT D'UN SUJET

(30) Priority: 07.01.2021 US 202163134662 P; 16.02.2021 EP 21157404
(43) Date of publication of application: 15.11.2023
(73) Proprietor: Signify Holding B.V., 5656 AE Eindhoven (NL)
(72) Inventor: PIJLMAN, Fetze, 5656 AE Eindhoven (NL); DEIXLER, Peter, 5656 AE Eindhoven (NL)
(74) Representative: Verweij, Petronella Daniëlle
(86) International application number: PCT/EP2022/050044
(87) International publication number: WO 2022/148733

(56) References cited:
- WO-A1-2020/169387
- CN-A- 106 774 825
- US-A1- 2008 077 015

## Description

### FIELD OF THE INVENTION

The invention relates to a detection system, a method and a computer program for detecting motion of a subject.

### BACKGROUND OF THE INVENTION

Today, using radiofrequency sensing for detecting motion of subjects using networks of a plurality of devices in home and office applications is of increasing interest. Documents CN106774825, US2008/077015 and WO2020/169387 disclose relevant prior art in this field.

The basic idea of radiofrequency is that network devices, including, for instance, luminaires, smart switches, smart application devices, etc. form a radio network by frequently exchanging messages, wherein the amplitude of the messages is then monitored and, for instance, compared to a baseline signal to determine the changes in the environment of a network device. The changes can be interpreted, for instance, as movement of persons, inactivity of a person, change in the status of objects, like open or closed doors, etc. However, the monitoring of the amplitudes of the exchanged messages is a complex and error-prone process often leading to false positive or negative results, for instance, to the false detection of persons in a room. Since radiofrequency sensing is often utilized for controlling functions of the network devices, for instance, lighting functions, inaccurate radiofrequency sensing results lead to disturbances in the application of the network devices and are highly undesired. Thus, it would be advantageous to provide a motion detection system for networks of network devices that allows for a higher accuracy.

### SUMMARY OF THE INVENTION

It is an object of the present invention to provide a detection system, a method and a computer program allowing for an improved detection of motion of a subject in an environment of the detection system.

In a first aspect of the present invention, a detection system for detecting motion of a subject by utilizing at least two devices adapted to send and receive radiofrequency signals is presented, wherein the system comprises a) a control unit for controlling the at least two devices such that at least one of the at least two devices is a sending device sending a radiofrequency signal with a sending signal frequency and such that at least one of the at least two devices is a receiving device receiving a radiofrequency signal that is indicative of reflections of the sent radiofrequency signal of the subject, b) a signal frequency providing unit for providing the sending signal frequency with which the radiofrequency signal has been sent, and c) a detection unit for detecting motion of the subject by performing a passive Doppler sensing based on the received radiofrequency signal and the provided sending signal frequency.

Since the detection unit is adapted for detecting motion of a subject by performing a passive Doppler sensing based on the received radiofrequency signal sent by one of the at least two devices and received by another of the at least two devices and the provided sending signal frequency, the passive Doppler sensing can be performed based on the knowledge of the sending signal frequency, i.e. without having to determine a sending signal frequency from the received radiofrequency signal itself. This allows for a high accuracy of detecting motion using radiofrequency signals. Thus, the detection system allows for an improved motion detection.

The detection system for detecting motion of the subject utilizes at least two devices that are adapted to send and receive radiofrequency signals. A subject can refer, for instance, to a living being or object, in particular, to a moving living being or object. A living being can refer to a human being or an animal and an object can refer to any object, for instance, to a robotic home appliance or a door. In a preferred embodiment, the detection system is adapted for detecting the motion of a human being in the vicinity of the at least two devices. Generally, simple objects like a robotic cleaner in a room, provide a simple Doppler signal, i.e. the objects have only one velocity. More complex objects or living beings comprise more complex Doppler signals, since often different parts of the complex objects or living beings move in different directions with different velocities. These more complex Doppler signals can also be regarded as Doppler signatures and allow to easily differentiate between different subject and their behavior.

Preferably, the two devices are network devices that are part of a network formed at least by the two network devices. However, the network can also comprise more than the two network devices, wherein in this case, the detection system can utilize all or only a part of the network devices as the at least two network devices for performing the motion detection. A network of network devices is generally formed by a communication of the network devices with each other, wherein the communication of the network devices of the network can be based on any known communication protocol, for instance, a WiFi communication protocol, a ZigBee communication protocol, a Bluetooth communication protocol, etc. It is thus preferred that the two devices being preferably network devices comprise a network device communication unit, wherein the network device communication unit is adapted to send and receive wireless signals, particularly radiofrequency signals, and/or wired signals. For instance, the network device communication unit can comprise a network device transceiver for receiving and transmitting radiofrequency signals, or a transmitter for transmitting radiofrequency signals and a receiver for receiving radiofrequency signals. In particular, the at least two devices can be smart network devices, i.e. any device comprising a communication unit for sending and receiving wireless signals, particularly radiofrequency signals, but which otherwise fulfills functions of a corresponding conventional device. For example, such a smart network device may be a smart home device, in which case a corresponding conventional function could be that of a conventional home device like a lighting device or a home appliance. In a preferred embodiment, the at least two devices refer to a smart light module, a smart plug or a smart switch.

The detection system can be a part of a network comprising the at least two devices, for instance, by being provided as software or hardware in one of the network devices or distributed over a plurality of network devices in communication with each other. However, the detection system can also be a standalone system or part of a device or devices that do not belong to a network but can communicate with at least one device being preferably part of a network. For instance, the detection system can be provided as software running on a handheld computational device or in another network that can communicate, for instance, via a gateway, with a network or at least one device being preferably part of a network. If the at least two devices are not part of a network, the detection system can be provided as part of one of the two devices, for instance, as software or hardware provided in one of the at least two devices, and can be adapted to communicate with both of the at least two devices via a wired or wireless communication protocol. Moreover, also in this case, the detection system can be a standalone system provided outside of the at least two devices, for instance, on a handheld computational device, a network solution, or any other computational device that is adapted to communicate with the at least two devices.

The control unit is adapted to control the at least two devices. Moreover, if more than two devices are provided, for instance, as part of a network of a plurality of network devices, the control unit can be adapted to control all or a part of these devices. Generally, the control unit can be adapted to control the at least two devices by sending control commands to the at least two devices that, when executed by the at least two devices, lead to a providing of functions of the at least two devices that are indicated by the control command.

The control unit is, in particular, adapted to control at least one of the at least two devices such that it acts as a sending device for sending a radiofrequency signal with a sending signal frequency. The sending signal frequency can refer to one frequency or to a range of frequencies. Moreover, the control unit can also be adapted to control the sensing device such that more than one radiofrequency signal each with different sensing signal frequency are sent. In particular, the sent radiofrequency signal is sent with a sending signal frequency lying in a predetermined signal frequency range, for instance, in the signal frequency range defined by the ZigBee standard lying at 2.4 GHz or in the frequency range of the WiFi standard communication lying in the frequency range around 2.5 GHz, 50 GHz or even 60 GHz. Within such a predefined frequency range, the control unit can be adapted to control the sending device such that it sends a radiofrequency signal with a specific sending signal frequency selected from the available range. However, the radiofrequency signal with the sending signal frequency sent by the sending device can also be predetermined, for instance, by the hardware of the sending device or by rules provided within the sending device such that in this case, the control unit only controls the sending device by causing the sending device to send the radiofrequency signal with the sending signal frequency indicated by the sending device itself. If the at least two devices refer to network devices, it is preferred that the sending network device is adapted to utilize its network device communication unit, in particular, a transmitter of the network device communication unit for transmitting the radiofrequency signals with the sending signal frequency. However, if the sending network device comprises a network device communication unit only adapted for wired network communication or for network communication in another range than radiofrequency range, the network device can comprise an additional unit for sending the radiofrequency signals. In a preferred embodiment, the radiofrequency signals sent by the sending device refer to communication signals within a network, i.e. to signals utilized for the communication between the network devices.

The control unit is further adapted for controlling at least one of the at least two devices for acting as a receiving device receiving a radiofrequency signal that is indicative of reflections of the sent radiofrequency signal of the subject. In particular, the control unit can be adapted to control the receiving device such that it monitors signals received in a predetermined frequency range in which reflections of the sent radiofrequency signal, in particular, when reflected by the subject, are expected. Preferably, if the receiving device refers to a network device, the receiving device utilizes the network device communication unit, in particular, a receiver of the network device communication unit, for receiving the reflected radiofrequency signal. However, the receiving device can also comprise a dedicated receiving unit that is not a part of the network device communication unit for receiving the reflected radiofrequency signal. The sending device and the receiving device generally do not refer to the same of the at least two devices. Thus, if only two devices are utilized, one of the two devices is the sending device and the other one of the two devices is the receiving device. In particular, the at least two devices are independent of each other and are not provided at the same location. Preferably, the sending device and the receiving device are provided with a certain predetermined distance to each other, wherein the distance is preferably greater than 1 m, more preferably more than 2 m. Thus, in a preferred embodiment, the controlling of the at least two devices by the control unit comprises a selection which of the at least two devices acts as a sending device and which of the at least two devices acts as a receiving device. In particular, if more than two devices are provided, i.e. a plurality of devices, the selection can comprise selecting one or more of the devices for acting as a sending device and selecting one or more of the devices for acting as a receiving device based, for instance, on the locations of the plurality of devices, on the characteristics of the plurality of devices, for instance, whether the devices comprise a radiofrequency sending and/or receiving unit, and/or on an availability of the devices, etc. Thus, the control unit is generally adapted to control a forming of device pairs, wherein one of the devices of such a device pair is regarded as a sending device for this device pair and the other device is regarded as a receiving device for this device pair. However, an overlapping of such pairs can also be contemplated such that, for instance, a device acts as sending device in one device pair and as receiving device in another device pair, or such that a sending device is the same for different device pairs, but the receiving device is different for each pair. In the last case, it can be regarded that the device pairs all comprising the same sending device form a device group. The device pairs or groups can be distinguished by distinguishing the signals sent by the sending devices of each device pair or group. For example, the control unit can be adapted to control each sending device of a device pair or group to send a radiofrequency signal at a different time or by utilizing a different sending signal frequency.

Generally, the control unit can be adapted control the sending device and/or the receiving device in a scan mode, wherein the scan mode allows for a directed scanning of at least a part of a sensing area. Preferably, in the scan mode the control unit is adapted to control the sensing device and/or receiving device to perform a directed sending and/or receiving. For example, the sensing device can be controlled to direct the radiofrequency signal, using known direction methods, subsequently to different areas of a room to scan the room systematically for the presence of motion. However, in other embodiments, the control unit can be adapted to control the sending and/or receiving device to perform an undirected sending, in particular, to send and/or receive signals in/from a plurality of directions at the same time.

Further, the detection system comprises a signal frequency providing unit for providing the sending signal frequency with which the radiofrequency signal has been sent. The signal frequency providing unit can be, for instance, a storage unit storing the sending signal frequency being, for instance, a predetermined sending signal frequency, or can be connected to a storage unit storing the sending signal frequency. The signal frequency providing unit can also be adapted as a receiving unit for receiving the sending signal frequency, for instance, from the control unit controlling the sending device or from the sending device itself and to then provide the received sending signal frequency. For example, the sending device can be adapted to monitor and provide the sending signal frequency of the radiofrequency signal sent by itself and to provide the sending signal frequency to a signal frequency providing unit for providing the same. In particular, the signal frequency providing unit can be part of the sending device and can then be adapted to communicate with the detection unit for providing the sending signal frequency. Generally, for all embodiments, if more than one sending device is controlled by the control unit, the signal frequency providing unit can be adapted to provide the sending signal frequency of each of the sending devices, in particular, if the sending devices use different sending signal frequencies.

The detection unit is then adapted to detect motion of the subject by performing a passive Doppler sensing based on the received radiofrequency signal and the provided sending signal frequency. Generally, passive Doppler sensing comprises performing a Doppler analysis on a received signal, wherein the receiver of the signal is not the same as the transmitter, i.e. wherein the transmitter is provided at a different location than the receiver of the signal. In this invention, the passive Doppler sensing is based not only on the received radiofrequency signal, but also based on knowledge on the sending signal frequency, i.e. on knowledge on the sent radiofrequency signal. This has the advantage that a complex and error-prone analysis of the received radiofrequency signal for extracting the sending signal frequency from the received radiofrequency signal can be omitted. In particular, based on the received radiofrequency signal and the provided sending signal frequency, a known Doppler analysis can be performed. A Doppler analysis is based on the principle that the frequency of a wave reflected from a moving subject will change depending on a velocity of the moving subject. Thus, by providing to the detection unit the received radiofrequency signal and the provided sending signal frequency, the detection unit can determine whether the received radiofrequency signal comprises at least a signal part with a frequency shifted with respect to the provided sending signal frequency that indicates that a motion of a subject is present. For detecting the motion of the subject based on the received radiofrequency signal and the provided sending signal frequency, the detection unit can be adapted to utilize any known software or hardware solution for extracting a frequency shift, i.e. for performing a Doppler analysis, from the received radiofrequency signal that is indicative of motion of a subject like signal frequency analysis methods, signal mixing methods, etc. Moreover, the detection unit is preferably adapted to differentiate between living beings and simple objects, for instance, based on the complexity of the result of the Doppler analysis. For example, for a simple object, the result comprises generally only one velocity, whereas for a living being, it is expected that the result leads to more than one determined velocity, i.e. to a velocity signature. The detection unit can then be adapted to further analyze the velocity signature, for instance, to determine an identity of the living being, an average velocity, a breathing motion, a movement direction, etc. based on the velocity signature. For example, the detection unit can be adapted to determine in a frequency spectrum of the received signal whether more than one Doppler shift can be found and can determine that this indicates a presence of a living being.

In an embodiment, the control unit is further adapted to control the at least two devices such that each of the at least two devices acts as a sending device sending each a radiofrequency signal with a different signal frequency and to control the at least two devices such that each acts as a receiving device to receive reflections of the sent radiofrequency signals of the subject corresponding to the sent radiofrequency signal of the respective other device, wherein the detection unit is adapted to perform the passive Doppler sensing based on the received radiofrequency signals. Thus, in this embodiment, the control unit is adapted to control the devices available for being utilized as sending and receiving devices such that the device pairs or groups formed as overlapping device pairs or groups. In particular, in this embodiment, a device group can be defined for each device that is capable to act as sending device, wherein the device group then comprises the device acting as sending device and all other devices that are capable of receiving the reflected radiofrequency signals of the sending device as receiving devices. Thus, a sending device capable of also receiving radiofrequency signals acts as sending device in its own group and as receiving device in other groups. The detection unit is then adapted to perform the passive Doppler sensing, i.e., the Doppler analysis, based on the received radiofrequency signals. For instance, the detection device can be adapted to perform the passive Doppler sensing for each of the groups independent from each other, preferably, based on the results of the Doppler analysis of all pairs of the group. However, the detection unit can also be adapted to utilize received radiofrequency signals from different groups for performing the passive Doppler sensing. For example, a detection unit can be adapted to compare the results of the passive Doppler sensing performed by different pairs of a group or by different groups with each other and to apply logical rules to the comparison to determine whether the results indeed refer to the motion of a subject, in particular, a human being, or are caused by other reasons, for instance, noise, vibrations of the device itself, etc. In particular, to decrease the influence of vibrations caused, for instance, by processes within the device or in the environment of the device on the detection of motion, it is preferred that the detection unit is adapted to compare the passive Doppler sensing results of all device pairs, wherein the receiving device of one pair is a sending device of the other pair and vice versa. Thus, passive Doppler sensing results based, preferably, on different sending signal frequencies and corresponding to substantially the same detection area, in particular, the area between the two devices forming the two device pairs, can be compared and logical rules can be applied to this comparison to determine whether the detection results are caused by a motion of a subject. For example, if the passive Doppler sensing results indicate the motion of a subject with different velocities, it is very unlikely that the detection results are caused by a moving subject, but, are very likely caused, for instance, by noise.

In an embodiment, the control unit is adapted to control the sending device to detect radiofrequency signals resulting from reflections of the sent radiofrequency signal sent by itself, and wherein the detection unit is adapted to monitor the detected radiofrequency signals and to detect motion of a subject further based on the monitored radiofrequency signals. In particular, the sending device is adapted to monitor radiofrequency signals resulting from reflections of the sent radiofrequency signal sent by itself that are not reflected by the subject. The signal frequency providing unit can then also be adapted to provide the detected radiofrequency signals to the detection unit. The detection unit is then adapted to monitor the detected radiofrequency signals detected by the sending device. The monitoring can comprise, for instance, a determining of changes in time of the detected radiofrequency signal. In particular, changes that occur suddenly, i.e. within a predetermined short time range, can be indicative of events that should be taken into account when detecting motion of the subject. Preferably, the detection unit is adapted to monitor the detected radiofrequency signal by monitoring the frequency occurring in the detected radiofrequency signal. In particular, it is preferred that the detection unit identifies in the frequency spectrum of the detected radiofrequency signal narrow frequency bands, i.e. signals in a frequency range smaller than a predetermined extent, preferably, an extend that refers according to the Doppler relation to a velocity variation of 20 cm/s, more preferably 10 cm/s. Such narrow frequencies are often a result of vibrations of the sending device itself or the environment of the sending device. Thus, when the application of the detection system is aimed at detecting a motion of a subject, preferably, a human being, it is preferred that the detection unit is adapted to use the identified narrow frequency range to filter out the identified narrow frequency range in the received radiofrequency signal, since it can be expected that also the received radiofrequency signal comprises frequencies, i.e. shows an excitation in a frequency spectrum, in the identified narrow frequency band that, however, is very likely not caused by the motion of a subject. The detection unit is then adapted to determine the motion of the subject by performing the passive Doppler sensing based on the filtered received radiofrequency signal.

According to the invention, the detection unit is adapted to determine an excitation in a frequency range in a spectrum of the received radiofrequency signal, wherein the excitation is substantially constant over a predetermined time period, and to perform the passive Doppler sensing based on the received radiofrequency signal by filtering out the frequency range in the spectrum of the received radiofrequency signal. Since it can be expected that motions of subjects, in particular, of human beings are not constant over a long time period, an excitation in a frequency range that is substantially constant over a long time period is very likely not caused by the movement of a subject, but is caused, for instance, by a vibration in the environment or by noise. Based on the application of the detection system, it is preferred that the predetermined time period is determined, for instance, on the time scales of motion that are expected for a motion of the subject that should be detected. For example, if the subject, for which motions should be detected, is a human being, the predetermined time period can refer to a few minutes, for instance, 3 or 4 minutes, since it is very unlikely that the motion of a human being, for instance, in a small room, does not change during such a time period. Thus, an excitation in the received radiofrequency signal that can be observed to be substantially constant, i.e. constant within a predetermined range of more than a few minutes, is very likely not caused by the motion of a subject and can be filtered out. In this context, the term substantially constant refers to the excitation being present at the same frequency or frequency range with an amplitude that does not fall below a noise threshold during the predetermined time period.

In a preferred embodiment, the detection unit can be adapted to determine an occurrence of an excitation in a frequency range that is narrower than a predetermined frequency range and to perform the passive Doppler sensing based on the received radiofrequency signal by filtering out the narrow frequency range in the spectrum of the received radiofrequency signal. In particular, if the motion of a subject that shall be detected refers to the motion of a human being, it can be expected that an excitation in a frequency range of a receiving radiofrequency signal caused by a moving human being is spread or smeared out over a wide frequency range, for instance, due to different parts of the body of a human being moved with different velocities like the arms and the legs moving with different velocities than the torso of a human being. Thus, excitations of the received radiofrequency signal occurring in a very narrow frequency range are very likely not caused by the motion of a human being or in fact any living being, but by vibrations of mechanical components in the environment of the sending or receiving device. Preferably, the predetermined frequency range refers according to the Doppler relation to a velocity variation of 20 cm/s, more preferably 10 cm/s. Thus, by filtering out excitations that are very likely caused by sources of motion that shall not be detected with respect to the application of the detection system or that refer to any kind of noise in the environment of the detection system, the accuracy of the motion detection, in particular, in view of the specific application of the detection system, can be increased.

In an embodiment, the detection unit is adapted to determine an I-channel and a Q-channel based on the received radiofrequency signal and to perform the passive Doppler sensing based on the I-channel and the Q-channel.

In particular, the I- and the Q-channel can be determined by the detection unit by multiplying the received radiofrequency signal with a signal comprising the provided sending signal frequency to determine the I-channel and by multiplying the received radiofrequency signal again with a signal comprising the provided sending signal frequency with one of these two signals being phase-shifted, for instance, by 90°, to determine the Q-channel. Preferably, the detection unit is then adapted to construct a complex signal from the Q-channel and the I-channel, wherein the I-channel refers to the imaginary part of the complex signal and the Q-channel to the real part of the complex signal. It is then preferred that the complex signal is utilized for detecting the motion of the subject. In particular, it is preferred that the frequency spectrum of the complex signal is determined by the detection unit, for instance, by performing a Fourier transform of the complex signal, and by detecting a motion of the subject based on the frequency spectrum of the complex signal. For example, the such constructed complex signal can show in the frequency spectrum positive and negative frequencies, wherein excitations in the positive frequencies indicate a motion with a motion component towards the receiving device while negative frequencies indicate a motion with a motion component away from the receiving device. However, the exact relationship between the motion and the determined frequencies depends on the phase-shift applied for constructing the Q-channel. Generally, independent of the exact phase-shift used, the construction of the complex signal allows an accurate determination, not only of a velocity of the motion of the subject, but also of a direction of the motion of the subject, at least with reference to the receiving device. Moreover, in the complex signal, in particular, in the frequency spectrum of the complex signal, motion not resulting from a subject, in particular, a human being, but, for instance, from vibrations in the environment of the devices, can be even more clearly distinguished. In particular, noise caused by vibrations in the environment leads to an excitation in the frequency spectrum of a complex signal in both the positive and negative frequencies, since vibrations comprise both motion components. In contrast thereto, a general movement of a subject like a human being comprises a more fixed direction and thus, only leads to an excitation either in the positive frequencies or in negative frequencies. Thus, it is preferred that the detection unit is adapted to identify an excitation in a frequency spectrum of the complex signal resulting from vibrations, for instance, due to the excitation being present both in the positive and negative frequencies, to filter the frequency range in which this excitation occurs out of the received radiofrequency signal and to base the Doppler analysis on the filtered received radiofrequency signal. In this context, it is noted that also a breathing motion of an otherwise non-moving person can be regarded as a vibration and thus can be detected utilizing the above approach by monitoring the positive and negative frequencies.

In an embodiment, the control unit is further adapted to control the sending device to send an additional radiofrequency signal with a signal frequency different from the sending signal frequency and to control the receiving device to receive an additional radiofrequency signal resulting from the reflection of the additional radiofrequency signal from the subject, wherein the detection unit is adapted to perform the passive Doppler sensing further based on the additional received radiofrequency signal. Thus, in this embodiment, each sending device controlled by the control unit is adapted to send two radiofrequency signals with different sending signal frequencies such that each receiving device controlled by the control unit can receive two radiofrequency signals that have been reflected from the subject based on the two different sent radiofrequency signals. Since the Doppler effect, i.e. the frequency shift due to motion in a reflected radiofrequency signal, is frequency-dependent for a moving subject, different frequency shifts in accordance with the principles of the Doppler effect can be found in the two different received radiofrequency signals. Generally, the sending signal frequency providing unit is in such cases adapted to provide both sending signal frequencies to the detection unit.

In a preferred embodiment, the detection unit is adapted to perform the passive Doppler sensing further based on the additional received radiofrequency signal by comparing the additional received radiofrequency signal with the received radiofrequency signal. Preferably, the comparison refers to a subtraction of the additional received radiofrequency signal from the received radiofrequency signal in the frequency domain, wherein the detection unit is adapted to detect motion based on the signal resulting from the subtraction. Generally, real motion of a subject leads, due to the principles of the Doppler effect, to an excitation in different frequency ranges of the two different received radiofrequency signals, whereas noise caused by, for instance, vibrations in the environment of the devices often leads to excitations in a frequency range that is independent of the sending signal frequency. Thus, by subtracting the two received radiofrequency signals from each other, frequency excitations lying in the same frequency range can be removed and the detection unit can then apply the Doppler analysis on the signal resulting from the subtraction that does not contain the excitation in the frequency range caused by noise. In another preferred embodiment, the detection unit can be adapted to perform the passive Doppler sensing by performing a Doppler analysis on both received radiofrequency signals independent of each other utilizing for each signal the provided respective sending signal frequency. The detection unit can then be adapted to compare the results of the two independent Doppler analyses to perform the passive Doppler sensing. For example, if a real moving subject is present, for instance, a human being, each of the two independent Doppler analyses will result in substantially the same determined velocity for the subject. However, if the results of the Doppler analyses are caused by noise, very likely different velocities are determined in each of the independent Doppler analyses indicating that the according signals are not caused by a real moving subject, but probably by noise or vibrations. Thus, the detection unit can be adapted to apply logical rules on the comparison of the two results from the two independent Doppler analyses to perform the passive Doppler sensing. The logical rules can be predetermined or can be learned rules that are learned by the detection unit, for instance, during a training phase, in which the detection unit is confronted with different environmental situations and a desired result of the passive Doppler sensing is also provided to the detection unit as input, for instance, provided by a user, and the detection unit is then adapted to learn, using known machine learning algorithms, logical rules that can be applied to achieve the desired results of the passive Doppler sensing. It is thus preferred that the comparison refers to performing a Doppler analysis on both signals independent of each other and to comparing the results of the Doppler analysis with respect to consistency.

In an embodiment, the performing of the Doppler analysis comprises applying a threshold filter to the received radiofrequency signal in the frequency domain. In particular, the received radiofrequency signal can be provided in the frequency domain, for instance, by applying a fast Fourier Transformation (FFT). In the frequency domain the received radiofrequency signal can optionally be further modified, for instance, by squaring the received radiofrequency signal. A threshold can then be predetermined, for instance, based on a calibration measurement or on experience, and provided to the threshold filter. The threshold filter then increases all signal parts of the received radiofrequency signal in the frequency domain that lie above the predetermined threshold and decreases all signal parts of the received radiofrequency signal in the frequency domain that lie beyond the threshold. The decreasing and increasing can be based on a predetermined value that is added or subtracted, respectively, can refer to applying a function, for instance, a proportional function, that depends on the difference of the signal part to the threshold, etc. The movement can then be determined based on the filtered received radiofrequency signal. This filtering allows for a more accurate determination of the movement.

In an embodiment, the control unit is adapted to control the sending device such that two radiofrequency signals with different sending signal frequencies being, for instance, 2.4 GHz and 5.8 GHz are utilized. In this embodiment, the performing of the Doppler sensing can comprise determining a belief vector for motion, for instance, the detection unit can comprise a dedicated logic that contains the belief vector for motion. Each component of this vector describes a probability for motion within a speed range relative to the receiving device. Preferably, the belief vector gets updated each 100 ms by analyzing the received radiofrequency signals that originate from the two different sending signals, here from the 2.4 GHz and 5.8 GHz frequency signals. For both sending signal frequencies, the detection unit can be adapted to perform as part of the Doppler analysis the following. A low pass filter having a predetermined cut-off frequency of, for instance, 120 Hz is provided to each of the received radiofrequency signals, after which the signal is sampled with a predetermined sample frequency, for instance, with 240 Hz. The detection unit is then adapted to, each 100 ms, use the last received 256 samples for computing a short fast Fourier transform resulting in a frequency spectrum. The frequency spectrum is then a set of bins where each bin contains a frequency range that can be mapped to a relative velocity range which can be mapped to the said belief vector for motion. Thus, the detection unit can be adapted to use the frequency spectrum subsequently for constructing a power spectrum by squaring the amplitudes of the frequency spectrum. For each bin in the power spectrum of which its value is above a chosen threshold, the corresponding components of the belief vector can then be increased. For each bin in the power spectrum of which its value is below a chosen threshold, the corresponding components of the belief vector can then be decreased. The detection unit is then adapted, after having updated the belief vector, to determine that a motion is detected when the largest value of all components exceeds 0.5, else the detection unit is adapted to determine that no motion is present.

In an embodiment, the detection unit is further adapted to perform radiofrequency sensing based on the amplitude of the received signal, wherein the detection unit is adapted to perform passive Doppler sensing alternatively or additionally to radiofrequency sensing based on a sensing result determined for at least one of the at least two devices and/or based on a device state of at least one of the at least two devices. In particular, the detection unit can be adapted to perform radiofrequency sensing using known radiofrequency algorithm as, for instance, described in WO 2020/043606 A1. Preferably, the detection unit is adapted to apply predetermined logical rules to decide based on the results of radiofrequency sensing whether passive Doppler sensing shall be performed alternatively or additionally to the radiofrequency sensing, for instance, for a predetermined time period. The predetermined logical rules can be adapted to the application of the system and can be situation-dependent. For example, the detection unit can be adapted to generally perform radiofrequency sensing, and if the detection unit determines that the results of the radiofrequency sensing might be unreliable, i.e. might not be conform with a predetermined quality criterion, the detection unit can be adapted to perform additionally a passive Doppler sensing as, for instance, described in the above embodiments to verify the result of the radiofrequency sensing. In another example, if at least one of the devices is in a sleep state, for instance, when it is not expected that people will be present in an area during the night, the detection unit can be adapted to apply instead of radiofrequency sensing passive Doppler sensing from time to time to detect motion in an area, wherein if motion is detected by the passive Doppler sensing, the detection unit can be adapted to additionally initiate the performing of radiofrequency sensing, for instance, by waking up the device, to get more information on the determined motion in the area.

In an embodiment, two pairs of devices are utilized for detecting motion, wherein the control unit is adapted to control each pair of devices such that each pair of devices comprises at least a sending and a receiving device, wherein the control unit is further adapted to control the sending and receiving devices such that a radiofrequency signal of a different frequency is used by the two pairs of devices for passive Doppler sensing, wherein the detection unit is adapted to perform the passive Doppler sensing for each pair of devices independently and to further detect a motion based on a comparison of the resulting detection results. In particular, it is preferred that the detected motion refers to minute movements or vibration of the subject. In this context, minute movements are defined as referring to movements that are small in size and/or time, wherein small in this context means below 10 cm in size and below 1 min in time. For instance, minute movements can refer to periodic movements of the body of a human being like breathing movements or heartbeat movements. Thus, in this embodiment, it is preferred that the system is specifically adapted such that a movement of a subject refers to the detection of a vibration movement or minute movement of the subject. In many of the above embodiments, it is preferred that these vibrations or minute movements are cancelled out of the motion detection of a subject. However, in other applications, it is advantageous to specifically detect the vibrations, for instance, in case of a machine monitoring for monitoring the function of a machine, wherein vibrations of the machine are often indicative of status changes within the machine. Accordingly, the principles discussed above with respect to removing the vibrations can now also be applied to adapt the detection unit to detect the vibrations or minute movements of a subj ect.

In an embodiment, a result of the motion detection is used for controlling a functionality of the devices. For example, it is preferred that the devices refer to lighting devices and that the lighting functionality is controlled based on the motion detection. However, also other functionalities can additionally or alternatively be controlled by the result of the motion detection performed by the system.

In an aspect of the present invention, a detection method for detecting motion of a subject by utilizing at least two devices adapted to send and receive radiofrequency signals is presented, wherein the method comprises a) controlling the at least two devices such that at least one of the at least two devices is a sending device sending a radiofrequency signal with a sending signal frequency and such that at least one of the at least two devices is a receiving device receiving a radiofrequency signal that is indicative of reflections of the sent radiofrequency signal of the subject, b) providing the sending signal frequency with which the radiofrequency signal has been sent, and c) detecting motion of the subject by performing a passive Doppler sensing based on the received radiofrequency signal and the provided sending signal frequency.

In another aspect of the present invention, a computer program product for detecting motion is presented, wherein the computer program product comprises program code means causing a detection system according to claim 1 to execute a detection method according to claim 13.

It shall be understood that the detection system as described above, the method as described above, and the computer program as described above, have similar and/or identical preferred embodiments, in particular, as defined in the dependent claims.

It shall be understood that a preferred embodiment of the present invention can also be any combination of the dependent claims or above embodiments with the respective independent claim.

These and other aspects of the invention will be apparent from and elucidated with reference to the embodiments described hereinafter.

### BRIEF DESCRIPTION OF THE DRAWINGS

In the following drawings:
Fig. 1 shows schematically and exemplarily two radiofrequency devices being utilized in a detection system controlling the radiofrequency devices, and
Fig. 2 shows schematically and exemplarily a detection method for detecting motion of a subject.

### DETAILED DESCRIPTION OF EMBODIMENTS

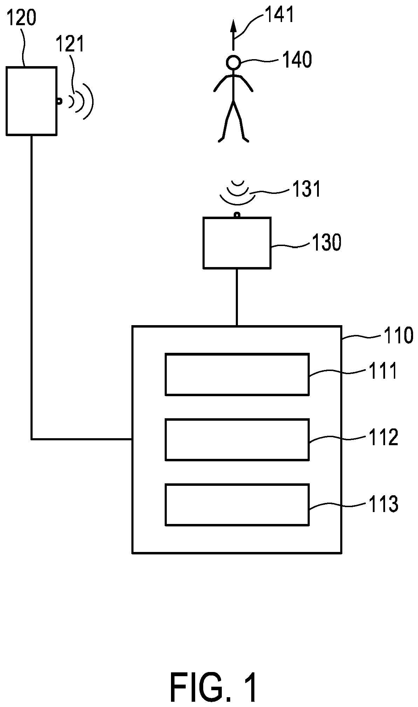

Fig. 1 shows schematically and exemplarily two radiofrequency devices 120, 130 and a detection system 110. Preferably, the radiofrequency devices 120, 130 are part of a network formed at least by the two radiofrequency devices 120, 130, wherein optionally the network comprises additional radiofrequency devices, not shown in Fig. 1, that can also be controlled by the detection system 110. However, for a better overview in the following the principles of the invention are described with respect to only the two radiofrequency devices 120, 130 shown in Fig. 1, whereas these explained principles can then be applied also to systems comprising more than the two radiofrequency devices 120, 130. It is in particular noted here that the two radiofrequency devices 120, 130 refer to completely different devices and are not located at the same location but are provided with a distance to each other that is preferably greater than 1 m, more preferably greater than 3 m.

The detection system 110 can be a stand-alone system that is in communicative contact with at least the two radiofrequency devices 120, 130 or can be provided as part of one of the at least two radiofrequency devices 120, 130, for instance, within a housing or as part of software and/or hardware provided in one of the at least two devices 120, 130. Preferably, if the at least two radiofrequency devices 120, 130 are part of a network, the detection system is also a part of the network, for instance, as part of one of the devices of the network or is distributed over a plurality of the devices of the network. In this case, a communication between the detection system 110 and the at least two devices 120, 130 and optionally other network devices can be part of the general network communication, i.e., messages from and to the detection system 110 are sent as part of the communication protocol used by the network.

The detection system 110 comprises a control unit 111, a signal frequency providing unit 112, and a detection unit 113. The control unit 111 is adapted to control the radiofrequency device 120 and the radiofrequency device 130. In particular, the control unit 111 is adapted to control the radiofrequency device 120 to act as a sending device sending a radiofrequency signal 121 with a sending signal frequency. Further, the control unit 111 is adapted to control the radiofrequency device 130 to act as a receiving device receiving a radiofrequency signal 131 that is a result of a reflection of the radiofrequency signal 121 from a subject, in this case a person 140 moving in a direction 141. Due to the Doppler effect the reflected signal 131 received by the radiofrequency device 130 comprises information on the movement 141 of the person 140, in particular, the signal 131 reflected from the person 140 experiences a frequency shift with respect to the sent radiofrequency signal 121. Preferably, the sent radiofrequency signal 121 is also sent as part of a general network communication, i.e. refers to a general network communication signal comprising, for instance, a message for another network device. However, the sent radiofrequency signal 121 can also be a dedicated signal only sent for motion detection.

The signal frequency providing unit 112 is adapted to provide the sending signal frequency of the sent radiofrequency signal 121. In particular, the detection system 110 is in communication with the radiofrequency device 120 acting as sending device such that the radiofrequency device 120 can provide the information on the sending signal frequency to the signal frequency providing unit 112. For example, the signal frequency providing unit 112 can be adapted to initiate a communication of the radiofrequency device 120, preferably, via the network communication signals, containing the sending signal frequency. However, in other embodiments the radiofrequency device 120 acting as sending device can be adapted to provide a sending signal frequency by itself to the signal frequency providing unit 112 or the sending signal frequency can be predetermined and stored on a storage from which the signal frequency providing unit 112 can read the sending signal frequency. The signal frequency providing unit 112 is then adapted to provide the sending signal frequency to the detection unit 113.

The detection unit 113 is adapted to detect motion of the person 140 by performing a passive Doppler sensing based on the received radiofrequency signal 131 and the provided sending signal frequency. The passive Doppler sensing comprises at least a Doppler analysis based on the received radiofrequency signal 131 and the provided sending signal frequency. For example, the detection unit 113 can be adapted, via hardware or software, to multiply an electronic representation of the received radiofrequency signal 131 with an electronic representation of a periodic signal with the sending signal frequency. Optionally, further a low pass filter can be provided on the resulting multiplied signal such that the filtered signal only comprises frequencies below a predetermined frequency threshold. Mathematically, the multiplication and low pass filtering of the signal can be regarded as determining a difference between the two input signals, wherein an excitation in a non-zero frequency range in the resulting signal is due to the Doppler effect of the moving person 140 on the sent radiofrequency signal 121. In a first approximation, the excitation frequency can be regarded as being directed proportional to the relative speed of the moving person 140. Thus, from the excitation frequency a movement 141 and even a velocity of the person 140 can be determined. However, the detection unit 113 can also be adapted to include more sophisticated analysis methods into the passive Doppler sensing. For example, the detection unit 131 can also utilize I- and Q-channels to determine the Doppler shift caused by the movement 141 of the person 140.

Some further more detailed embodiments and applications of the detection system 110 will be described in the following. In the following embodiments, the at least two devices are part of a network of network devices that can be controlled by the control unit. Generally, the network can be based on different network communication protocols like WiFi, Bluetooth, Zigbee, etc.

In an embodiment, the network refers, for instance, to a WiFi, e.g. 2.4 GHz, network. In this case, the control unit can be adapted to control the network devices to send radiofrequency signals in an ultra-wideband frequency range on frequencies that do not overlap. This non-overlap can be realized by using the available channels but also by using complete different frequencies such as 2.4 GHz and 60 GHz of the WiFi communication protocol. Moreover, the detection unit can be provided with the sending signal frequency, i.e. used frequency channels, of all network devices. However, if the detection unit is part of every network device, the detection unit of the network device can be provided with only the sending signal frequency of neighboring network devices. Using passive Doppler sensing, the detection unit, optionally each detection unit of each network device independently, can then try to sense motion, for instance, by looking for non-zero frequency components in a multiplication of the received radiofrequency signal and a signal with corresponding sending signal frequency. Moreover, the detection unit can be adapted to identify possible noise sources like vibrations that can manifest, for instance, as narrow frequency range excitations in the received radiofrequency signal and are often caused by mechanical vibrations or electromagnetic interference. The identification of the noise in the received radiofrequency signal can be based on a monitoring of a low frequency spectrum of the sent radiofrequency signal that has not been reflected by the subject. In particular, each network device can also receive the radiofrequency signals resulting from its own sent signal that has not been reflected by the subject. Excited frequencies in these monitored signals with a narrow frequency range that often occur suddenly in the monitored signal can then be identified. Such frequencies can then consequently be ignored by the detection unit in the passive Doppler sensing. Additionally or alternatively, the monitoring of vibrations can be realized by the detection unit by analyzing the Doppler frequency spectrum of the received radiofrequency signal. A narrow frequency excitation that is constant, i.e. not moving in frequency spectrum, over a time of more than, for instance, 500 ms, can then be identified as vibration and filtered out for the Doppler sensing. Moreover, the detection unit can be adapted to employ two channels that are phase shifted, for instance, by 90°, i.e. channels cl(t) and c2(t), referring to the I- and Q-channels. From these channels the detection unit can be adapted to construct a new signal s(t)=cl(t)+ i c2(t), with i being the imaginary number. Based on the complex Fourier transform of this new signal, the Doppler sensing can be performed, for example, by subtracting a positive frequency spectrum from a negative frequency spectrum of the complex Fourier transform and performing a Doppler analysis on the result.

In an embodiment, the network can further be adapted to perform radiofrequency sensing, for instance, by exchanging short network messages and by monitoring the changes in amplitude of these messages that are indicative of time variations in the environment. In such a case, the detection can be adapted to double confirm the results of the radiofrequency sensing through the temporary switch of a passive Doppler sensing. This has the benefit that the default mode of operation of the network devices is standby power friendly.

In an embodiment, the network can also be adapted to make use of 5.8 GHz multichannel. For example, the control unit can be adapted to provide a trigger signal or to cause one of the network devices to provide a trigger signal, wherein based on the trigger signal the network devices are adapted to send two radiofrequency signals, one in the 5.8 GHz range and one in the 24 GHz range. The detection unit can then be adapted to analyze both spectra of the received signals and to subtract overlapping parts. The insight in this embodiment is that motion signals generally appear in different parts of the spectra and thus will still be present in the resulting spectrum. In contrast thereto, a vibration occurs in both spectra at similar frequencies and thus will be cancelled out by subtraction in the resulting spectrum. Generally, the detection device can be adapted to assign a lower sending signal frequency, e.g. 2.4 GHz, to sending devices for which it is expected that they suffer more from vibrations, for example, due to being located near a vibration source, and to assign the higher sending signal frequency, e.g. 60 GHz, to sending devices for which it is expected that they suffer less from vibrations.

The presence of vibrations or singular spikes in the received signal sometimes correlates with periods in which temperature changes and is a result of the mechanical elements of the network devices expanding and/or contracting. If the network devices comprise a lighting functionality, this particularly happens when the light is switch on or switched off. Generally, false positives detections of motion or presents due to vibrations are not very problematic when the light has just switched on, since there should be someone present anyway. However, when the light has just switched off, such detected false positives are not desired. Thus, to avoid false positive detection either in the passive Doppler sensing results or the radiofrequency sensing results, the detection unit can be adapted to start with either of the two sensing approaches and then additionally perform the other only during the difficult switch off time period, e.g. 0 to 30 min after light has been switch off, for maximizing the robustness of the detection against vibrations and achieve a quite standby power system.

In an embodiment, instead of trying to avoid the influence of vibrations, it can be desired to detect specifically vibrations of a subject. In this case, the detection unit can be adapted to control a sending device to send a radiofrequency signal with sending signal frequency that is sensitive to mechanical vibrations of either the sending device itself and/or subjects in its field of view. For instance, a lighting device can thus be used to monitor vibrations of a heating, ventilation and air conditioning (HVAC) gear close by an office ceiling. In this example, suddenly increased vibration of the HVAC equipment can lead to vibrations of the lighting device in the vicinity and indicates an imminent HVAC equipment failure. The above described system can then be employed to monitor such vibration events. In another exemplary application, the detection device can be adapted to employ passive Doppler sensing, optionally with additional radiofrequency sensing, to monitor the active times of machinery, like a conveyor belt, and also, for instance, to monitor changes in the vibration patterns of the machinery that are indicative of imminent damage to components of the machinery. Based on such monitoring a maintenance can be proactively scheduled to avoid more severe damages to the machinery and unplanned downtime.

In an embodiment, the control unit can be adapted to select sending and receiving devices such that two different pairs of devices are assigned to monitor an environment for movement, wherein both device pairs have overlapping sensing fields of view. The control unit can, for instance, control the first pair such that it uses a sending signal frequency in the 5 GHz WiFi range, while controlling the second pair such that it uses a sending signal frequency on the 60 GHz WiFi range. The detection unit can then be adapted to perform passive Doppler sensing based on the received signals of the two pairs, for instance, in order to perform fall-detection of either persons or parcels in, for instance, a warehouse. A fall is generally characterized by an acceleration of 1 g, followed by a deceleration of about -5 g. By monitoring a target area simultaneously with two different frequencies, it is possible to monitor fall detection and to monitor presence of people. For example, the detection unit can be adapted to utilize the 5.8 GHz signals for fall detection, since a falling of objects leads often to large frequency differences which are visible in the spectrum of the receiving signal of the 5.8 GHz device pair. For monitoring a presence of a human being, for instance, through sensing breathing, the detection unit can be adapted to utilize the higher frequencies which tend to offer more high resolution frequency spectra. Moreover, when rigid objects fall to the floor, they will briefly vibrate after hitting the floor hard. Thus, the detection unit can also be adapted to monitor the vibrations of, for instance, just-fallen objects from a warehouse shelf/forklift to assess how hard the object has hit the floor.

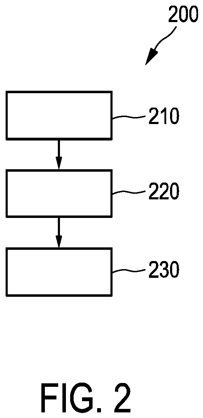

Fig. 2 shows schematically and exemplarily a method for detecting a movement of a subject. The method 200 comprises a first step 210 of controlling at least two radiofrequency devices, for instance, radiofrequency devices 120, 130 shown in Fig. 1, such that at least one of the at least two devices is a sending device sending a radiofrequency signal, for instance, radiofrequency signal 121, with a sending signal frequency and such that at least one of the at least two radiofrequency devices 120, 130 is a receiving device receiving a radiofrequency signal, for instance, radiofrequency signal 131, that results from reflections of a sent radiofrequency signal of the subject. In a further step 220, the method comprises providing the sending signal frequency with which the radiofrequency signal has been sent, for instance, for being used in the next step 230. In step 230, the motion of the subject is detected by performing a passive Doppler sensing based on the received radiofrequency signal and the provided sending signal frequency, for instance, in accordance with the principles explained above.

In the following some general principles will be explained. Radiofrequency sensing is a technology of increasing interest. The basic idea is that a system of wireless radiofrequency devices exchanges messages of which the amplitude is monitored. Any change in amplitude is an indication of a change in an environment that is close to the transmitter and receiver radiofrequency devices. Radiofrequency sensing can utilize standard wireless radiofrequency devices. For example, ZigBee devices mostly use 2.4 GHz signals, though the ZigBee standard also can be implemented at 868 MHz. Many modern WiFi devices now use both 2.4 GHz and 5 GHz signals and in a few years will even use 60 GHz WiFi signals.

In recent years, so-called passive Doppler sensing has been developed. This technology is challenging as one needs to recognize in the signal both the signal from the moving object as well as the carrier frequency. However, an advantage of using passive Doppler sensing is the richness of information that can be achieved compared to standard radiofrequency sensing.

Generally, the higher a frequency, i.e. the shorter the wavelength, the more susceptible becomes a sensing system employing radiofrequency signals for mechanical vibrations of the sending or receiving devices. For example, mechanical vibrations from, for instance, a louvre of a luminaire being a network device can critically influence mass-market 5.8 GHz radiofrequency sensor performance. Hence, there is a need to carefully design the sensing algorithms with robustness to vibrations in mind when utilizing radiofrequency sensing. Moreover, it is known that traditional preventative maintenance resolves 18 % of issues on machinery, while 82 % of machines break down due to random or unknown factors. Prior art also teaches that the 82 % unknown factors are best identified through the consistent monitoring of machinery and addressed with predictive maintenance. It is known that many times the motors within the machines themselves are early indicators for when there is a risk for unplanned downtime. It is known that especially vibration and temperature sensors reveal the status of a motor, e.g. a motor for a conveyor belt. To track a motor operation 24 hours a day, it is suggested based on the above described invention to employ vibration sensing utilizing a detection system as described above to determine, for instance, an average total use time of a machinery which helps to understand what the threshold is for preventative or scheduled maintenance. For instance, with the obtained data it can be derived that a threshold of 2500 hours of use is still acceptable, but that a preventative maintenance should be scheduled at 2000 hours to limit the risk of unplanned downtime. In particular, a detection system as described above can be utilized for monitoring the vibration of a machinery without the need to directly attach a vibration sensor to the machinery. By carefully monitoring vibration frequencies and patterns, for example, a baseline for the status of a motor can be established and ultimately the motor health status can be estimated. For instance, for many months, a motor's vibration level remains in line with a predetermined baseline such that no maintenance is necessary. However, eventually the vibration frequency and/or amplitude starts to increase, for example, because of a motor part that has worn thin. Based on the difference to the baseline, it can be determined that the motor part needs to be replaced soon.

In another application, the detection system can be employed for automatic lighting control. The requirements for automatic lighting control are often very stringent. Switching on the light when a person enters should be rapid, but switching on the light when nobody is there, i.e. false positive, is highly undesired. In some applications, the actuation of the light also has a safety element, for instance, in a multi-aisle warehouse, switching on the light in an intersection between two isles warns that a forklift is approaching the intersection. Moreover, if radiofrequency sensing is applied for the lighting control in most applications, the sensing performance depends on the light state itself, e.g. false positives are highly undesired when the light is off as it leads to a very visual misfunctioning, i.e. malfunctioning, of the system. The above described invention allows to overcome these problems by providing in addition or alternative to the radiofrequency sensing passive Doppler sensing for controlling the lighting system.

Preferably, for the passive Doppler sending two different sending signal frequencies, for instance, 2.4 GHz WiFi and 5 GHz WiFi, are employed to eliminate false triggers due to vibrations as described in detail above. In particular, in applications in which a strong influence of vibrations is expected, like, where an office troffer is installed in a system ceiling close to a HVAC duct or a suspended luminaire in a warehouse is placed near heavy machinery, using two sending signal frequencies can be advantageous. Moreover, besides the vibration of the sending and/or receiving devices themselves, also vibrations of objects within the field of view of the sensing system can lead to false triggers. Also this can be addressed by utilizing two different frequencies for the sending signal frequency as described above.

Moreover, to allow for a decrease of the influence of vibrations, the detection system as described above can be adapted to control a network device system, for example, a lighting system, and can be adapted to apply passive Doppler sensing that ignores narrow frequency excitations if the network device system is in an off state. In this context, the detection unit can be adapted to apply a filtering of the received signals, for example, through the use of I- and Q-channels and to subtract positive from negative frequencies. Preferably, in a lighting system the passive Doppler sensing is applied by the detection unit in a light-off state and/or is only applied after a radio frequency sensing has observed a trigger like the presents of a person. In an embodiment, two different frequency carriers, i.e. two different sending signal frequencies, are used for the passive Doppler sensing and a comparison between the two signals is made for identifying vibrations present either in the devices or in the field of view of the sensing. The comparison can be made in the frequency domain and the resulting spectra can be subtracted. Vibrations occur at a frequency that does not depend on the carrier frequency, whereas the Doppler effect depends on the carrier frequency. Thus, by subtracting the resulting spectra, the vibrations can be removed from the signal but real motions are kept. Additionally or alternatively, the comparison can be made in the time domain by comparing the energy present in both channels. Only if both channels are sufficiently above a predetermined threshold, a further motion analysis is performed.

Preferably, the passive Doppler sensing is performed during a cool down phase of a device. During a cool down phase of a device microwave carrier frequencies emitted by the device can interfere with nearby radiofrequency sensors due to a carrier shift. Hence, while a first pair of lights is meant to have a different carrier frequency, i.e. sending signal frequency, then an adjacent second pair of lights performing sensing in a radiofrequency range, due to the temperature change of the first pair of lights, the carrier frequency of the first pair of lights changes and may become the same frequency as that of the second pair of lights. Hence, the sensing of the first and second pair of lights can interfere with each other. However, this can only happen to one channel, i.e. sending signal frequency, at the time. Thus, performing passive Doppler sensing based on a plurality of different sending signal frequencies can help to identify and avoid this problem. This embodiment can also be applied to a turn on phase of a device.

In an embodiment, it is preferred that a sending of radiofrequency signals is switched off if a function of the device, for instance, the light, has been turned off for a significant amount of time, e.g. more than 30 min, to save energy as well as to reduce unwanted wireless smog.

In the claims, the word "comprising" does not exclude other elements or steps, and the indefinite article "a" or "an" does not exclude a plurality.

A single unit or device may fulfill the functions of several items recited in the claims. The mere fact that certain measures are recited in mutually different dependent claims does not indicate that a combination of these measures cannot be used to advantage.

Procedures like the controlling of the at least two devices, the providing of the sending radiofrequency signal, and the detection of motion, et cetera, performed by one or several units or devices can be performed by any other number of units or devices. These procedures can be implemented as program code means of a computer program and/or as dedicated hardware.

A computer program product may be stored/distributed on a suitable medium, such as an optical storage medium or a solid-state medium, supplied together with or as part of other hardware, but may also be distributed in other forms, such as via the Internet or other wired or wireless telecommunication systems.

Any reference signs in the claims should not be construed as limiting the scope.

The invention relates to a detection system for detecting motion of a subject by utilizing at least two devices adapted to send and receive radiofrequency signals. The system comprises a control unit for controlling the at least two devices such that at least one of the at least two devices is a sending device sending a radiofrequency signal with a sending signal frequency and such that at least one of the at least two devices is a receiving device receiving a radiofrequency signal that is indicative of reflections of the sent radiofrequency signal of the subject, a signal frequency providing unit for providing the sending signal frequency with which the radiofrequency signal has been sent, and a detection unit for detecting motion of the subject by performing a passive Doppler sensing based on the received radiofrequency signal and the provided sending signal frequency.

## Claims

1. A detection system for detecting motion (141) of a subject (140) by utilizing at least two devices (120, 130) adapted to send and receive radiofrequency signals (121, 131), wherein the system (110) comprises:
a control unit (111) for controlling the at least two devices (120, 130) such that at least one of the at least two devices (120, 130) is a sending device (120) sending a radiofrequency signal (121) with a sending signal frequency and such that at least one of the at least two devices (120, 130) is a receiving device (130) receiving a radiofrequency signal (131) that is indicative of reflections of the sent radiofrequency signal of the subject (140),
a signal frequency providing unit (112) for providing the sending signal frequency with which the radiofrequency signal (121) has been sent, and
a detection unit (113) for detecting motion (141) of the subject (140) by performing a passive Doppler sensing based on the received radiofrequency signal (131) and the provided sending signal frequency,
**characterized in that** the detection unit (113) is adapted to determine an excitation in a frequency range in a spectrum of the received radiofrequency signal (131), wherein the excitation is substantially constant over a predetermined time period, and to perform the passive Doppler sensing based on the received radiofrequency signal (131) by filtering out the frequency range in the spectrum of the received radiofrequency signal (131).

2. The detection system according to claim 1, wherein the control unit (111) is further adapted to control the at least two devices (120, 130) such that each of the at least two devices (120, 130) acts as a sending device (120) sending each a radiofrequency signal (121) with a different signal frequency and to control the at least two devices (120, 130) such that each acts as a receiving device (130) to receive a radiofrequency signal (131) that is indicative of reflections of the sent radiofrequency signal (121) of the subject (140) corresponding to the sent radiofrequency signal (121) of the respective other device, wherein the detection unit (113) is adapted to perform the passive Doppler sensing based on the received radiofrequency signals (131).

3. The detection system according to any of claims 1 and 2, wherein the control unit (111) is adapted to control the sending device (120) to detect radiofrequency signals resulting from reflections of the sent radiofrequency signal (121) sent by itself, and wherein the detection unit (113) is adapted to monitor the detected radiofrequency signals and to detect motion (141) of a subject (140) further based on the monitored radiofrequency signals.

4. The system according to any of the preceding claims, wherein the detection unit (113) is adapted to determine an I-channel and a Q-channel based on the received radiofrequency signal (131) and to perform the passive Doppler sensing based on the I-channel and the Q-channel.

5. The detection system according to any of the preceding claims, wherein the control unit (111) is further adapted to control the sending device (120) to send an additional radiofrequency signal with a signal frequency different from the sending signal frequency and to control the receiving device (130) to receive an additional radiofrequency signal resulting from the reflection of the additional radiofrequency signal from the subject (140) and wherein the detection unit (113) is adapted to perform the passive Doppler sensing further based on the additional received radiofrequency signal.

6. The detection system according to claim 5, wherein the detection unit (113) is adapted to perform the passive Doppler sensing further based on the additional received radiofrequency signal by comparing the additional received radiofrequency signal with the received radiofrequency signal (131).

7. The system according to claim 6, wherein the comparison refers to a subtraction of the additional received radiofrequency signal from the received radiofrequency signal (131) in the frequency domain, wherein the detection unit (113) is adapted to detect motion (141) based on the signal resulting from the subtraction.

8. The system according to claim 6, wherein the comparison refers to performing a Doppler analysis on both signals independent of each other and to comparing the results of the Doppler analysis with respect to consistency.

9. The system according to any of the preceding claims, wherein the detection unit (113) is further adapted to perform radiofrequency sensing based on the amplitude of the received signal and wherein the detection unit (113) is adapted to perform passive Doppler sensing alternatively or additionally to radiofrequency sensing based on a sensing result determined for at least one of the at least two devices (120, 130) and/or based on a device state of at least one of the at least two devices (120, 130).

10. The system according to any of the preceding claims, wherein two pairs of devices are utilized for detecting motion, wherein the control unit (111) is adapted to control each pair of devices such that each pair of devices comprises at least a sending and a receiving device, wherein the control unit (111) is further adapted to control the sending and receiving devices such that a radiofrequency signal of a different frequency is used by the two pairs of devices for passive Doppler sensing, wherein the detection unit (113) is adapted to perform the passive Doppler sensing for each pair of devices independently and to further detect a motion based on a comparison of the resulting detection results.

11. The system according to claim 10, wherein the detected motion refers to minute movements or vibration of the subject.

12. The system according to any of the preceding claims, wherein a result of the motion detection is used for controlling a functionality of the devices.

13. A detection method for detecting motion of a subject by utilizing at least two devices adapted to send and receive radiofrequency signals, wherein the method (200) comprises:
controlling (210) the at least two devices such that at least one of the at least two devices is a sending device sending a radiofrequency signal with a sending signal frequency and such that at least one of the at least two devices is a receiving device receiving a radiofrequency signal that is indicative of reflections of the sent radiofrequency signal of the subject,
providing (220) the sending signal frequency with which the radiofrequency signal has been sent, and
detecting (230) motion of the subject by performing a passive Doppler sensing based on the received radiofrequency signal and the provided sending signal frequency,
**characterized in that** the method further comprises determining an excitation in a frequency range in a spectrum of the received radiofrequency signal (131), wherein the excitation is substantially constant over a predetermined time period, and to perform the passive Doppler sensing based on the received radiofrequency signal (131) by filtering out the frequency range in the spectrum of the received radiofrequency signal (131).

14. A computer program product for detecting motion, wherein the computer program product comprises program code means causing a detection system according to claim 1 to execute a detection method according to claim 13.

## Patentansprüche

1. Erkennungssystem zum Erkennen einer Bewegung (141) eines Subjekts (140) durch Nutzung von mindestens zwei Vorrichtungen (120, 130), die angepasst sind, um Hochfrequenzsignale (121, 131) zu senden und empfangen, wobei das System (110) umfasst:
eine Steuereinheit (111) zum Steuern der mindestens zwei Vorrichtungen (120, 130) derart, dass mindestens eine der mindestens zwei Vorrichtungen (120, 130) eine Sendevorrichtung (120) ist, die ein Hochfrequenzsignal (121) mit einer Sendesignalfrequenz sendet, und derart, dass mindestens eine der mindestens zwei Vorrichtungen (120, 130) eine Empfangsvorrichtung (130) ist, die ein Hochfrequenzsignal (131) empfängt, das Reflexionen des gesendeten Hochfrequenzsignals des Subjekts (140) anzeigt,
eine Signalfrequenzbereitstellungseinheit (112) zum Bereitstellen der Sendesignalfrequenz, mit der das Hochfrequenzsignal (121) gesendet wurde, und
eine Erkennungseinheit (113) zum Erkennen der Bewegung (141) des Subjekts (140) durch Durchführen einer passiven Doppler-Erfassung basierend auf dem empfangenen Hochfrequenzsignal (131) und der bereitgestellten Sendesignalfrequenz,
**dadurch gekennzeichnet, dass** die Erkennungseinheit (113) angepasst ist, um eine Anregung in einem Frequenzbereich in einem Spektrum des empfangenen Hochfrequenzsignals (131) zu bestimmen, wobei die Anregung über einen zuvor bestimmten Zeitraum im Wesentlichen konstant ist, und um die passive Doppler-Erfassung basierend auf dem empfangenen Hochfrequenzsignal (131) durch Herausfiltern des Frequenzbereichs in dem Spektrum des empfangenen Hochfrequenzsignals (131) durchzuführen.

2. Erkennungssystem nach Anspruch 1, wobei die Steuereinheit (111) ferner angepasst ist, um die mindestens zwei Vorrichtungen (120, 130) derart zu steuern, dass jede der mindestens zwei Vorrichtungen (120, 130) als eine Sendevorrichtung (120) fungiert, die jeweils ein Hochfrequenzsignal (121) mit einer unterschiedlichen Signalfrequenz sendet, und um die mindestens zwei Vorrichtungen (120, 130) derart zu steuern, dass jede als eine Empfangsvorrichtung (130) fungiert, um ein Hochfrequenzsignal (131) zu empfangen, das Reflexionen des gesendeten Hochfrequenzsignals (121) des Subjekts (140) anzeigt, das dem gesendeten Hochfrequenzsignal (121) der jeweils anderen Vorrichtung entspricht, wobei die Erkennungseinheit (113) angepasst ist, um die passive Doppler-Erfassung basierend auf den empfangenen Hochfrequenzsignalen (131) durchzuführen.

3. Erkennungssystem nach einem der Ansprüche 1 und 2, wobei die Steuereinheit (111) angepasst ist, um die Sendevorrichtung (120) zu steuern, um Hochfrequenzsignale zu erkennen, die aus Reflexionen des Hochfrequenzsignals (121) resultieren, das allein gesendet wird, und wobei die Erkennungseinheit (113) angepasst ist, um die erkannten Hochfrequenzsignale zu überwachen und basierend auf den überwachten Hochfrequenzsignalen eine Bewegung (141) eines Subjekts (140) zu erkennen.

4. System nach einem der vorstehenden Ansprüche, wobei die Erkennungseinheit (113) angepasst ist, um einen I-Kanal und einen Q-Kanal basierend auf dem empfangenen Hochfrequenzsignal (131) zu bestimmen und um die passive Doppler-Erfassung basierend auf dem I-Kanal und dem Q-Kanal durchzuführen.

5. Erkennungssystem nach einem der vorstehenden Ansprüche, wobei die Steuereinheit (111) ferner angepasst ist, um die Sendevorrichtung (120) zu steuern, um ein zusätzliches Hochfrequenzsignal mit einer Signalfrequenz zu senden, die sich von der Sendesignalfrequenz unterscheidet, und um die Empfangsvorrichtung (130) zu steuern, um ein zusätzliches Hochfrequenzsignal zu empfangen, das aus der Reflexion des zusätzlichen Hochfrequenzsignals von dem Subjekt (140) resultiert, und wobei die Erkennungseinheit (113) angepasst ist, um die passive Doppler-Erfassung ferner basierend auf dem zusätzlichen empfangenen Hochfrequenzsignal durchzuführen.

6. Erkennungssystem nach Anspruch 5, wobei die Erkennungseinheit (113) angepasst ist, um die passive Doppler-Erfassung ferner basierend auf dem zusätzlich empfangenen Hochfrequenzsignal durch Vergleichen des zusätzlich empfangenen Hochfrequenzsignals mit dem empfangenen Hochfrequenzsignal (131) durchzuführen.

7. System nach Anspruch 6, wobei sich der Vergleich auf eine Subtraktion des zusätzlich empfangenen Hochfrequenzsignals von dem empfangenen Hochfrequenzsignal (131) in dem Frequenzbereich bezieht, wobei die Erkennungseinheit (113) angepasst ist, um die Bewegung (141) basierend auf dem Signal zu erkennen, das aus der Subtraktion resultiert.

8. System nach Anspruch 6, wobei sich der Vergleich auf das Durchführen einer Doppler-Analyse beider Signale unabhängig voneinander und auf dem Vergleichen der Ergebnisse der Doppler-Analyse hinsichtlich einer Konsistenz bezieht.

9. System nach einem der vorstehenden Ansprüche, wobei die Erkennungseinheit (113) ferner angepasst ist, um eine Radiofrequenzerfassung basierend auf der Amplitude des empfangenen Signals durchzuführen, und wobei die Erkennungseinheit (113) angepasst ist, um eine passive Doppler-Erfassung alternativ oder zusätzlich zu der Radiofrequenzerfassung basierend auf einem Erfassungsergebnis, das für mindestens eine der mindestens zwei Vorrichtungen (120, 130) bestimmt wird, und/oder basierend auf einem Vorrichtungszustand von mindestens einem der mindestens zwei Vorrichtungen (120, 130) durchzuführen.

10. System nach einem der vorstehenden Ansprüche, wobei zwei Vorrichtungspaare zum Erkennen der Bewegung genutzt werden, wobei die Steuereinheit (111) angepasst ist, um jedes Vorrichtungspaar derart zu steuern, dass jedes Vorrichtungspaar mindestens eine Sende- und eine Empfangsvorrichtung umfasst, wobei die Steuereinheit (111) ferner angepasst ist, um die Sende- und die Empfangsvorrichtung derart zu steuern, dass ein Hochfrequenzsignal einer unterschiedlichen Frequenz durch die zwei Vorrichtungspaare für die passive Doppler-Erfassung verwendet wird, wobei die Erkennungseinheit (113) angepasst ist, um die passive Doppler-Erfassung für jedes Vorrichtungspaar unabhängig durchzuführen und um ferner eine Bewegung basierend auf einem Vergleich der resultierenden Erkennungsergebnisse zu erkennen.

11. System nach Anspruch 10, wobei sich die erkannte Bewegung auf winzige Bewegungen oder Vibrationen des Subjekts bezieht.

12. System nach einem der vorstehenden Ansprüche, wobei ein Ergebnis der Bewegungserkennung zum Steuern einer Funktionalität der Vorrichtungen verwendet wird.

13. Erkennungsverfahren zum Erkennen der Bewegung eines Subjekts durch Nutzung von mindestens zwei Vorrichtungen, die angepasst sind, um Hochfrequenzsignale zu senden und empfangen, wobei das Verfahren (200) umfasst:
Steuern (210) der mindestens zwei Vorrichtungen derart, dass mindestens der mindestens zwei Vorrichtungen eine Sendevorrichtung ist, die ein Hochfrequenzsignal mit einer Sendesignalfrequenz sendet, und derart, dass mindestens eine der mindestens zwei Vorrichtungen eine Empfangsvorrichtung ist, die ein Hochfrequenzsignal empfängt, das Reflexionen des gesendeten Hochfrequenzsignals des Subjekts anzeigt,
Bereitstellen (220) der Sendesignalfrequenz, mit der das Hochfrequenzsignal gesendet wurde, und
Erkennen (230) der Bewegung des Subjekts durch Durchführen einer passiven Doppler-Erfassung basierend auf dem empfangenen Hochfrequenzsignal und der bereitgestellten Sendesignalfrequenz,
**dadurch gekennzeichnet, dass** das Verfahren ferner das Bestimmen einer Anregung in einem Frequenzbereich in einem Spektrum des empfangenen Hochfrequenzsignals (131) umfasst, wobei die Anregung über einen zuvor bestimmten Zeitraum im Wesentlichen konstant ist, und um die passive Doppler-Erfassung basierend auf dem empfangenen Hochfrequenzsignal (131) durch Herausfiltern des Frequenzbereichs in dem Spektrum des empfangenen Hochfrequenzsignals (131) durchzuführen.

14. Computerprogrammprodukt zum Erkennen der Bewegung, wobei das Computerprogrammprodukt Programmcodemittel umfasst, die ein Erkennungssystem nach Anspruch 1 veranlassen, ein Erkennungsverfahren nach Anspruch 13 auszuführen.

## Revendications

1. Système de détection pour la détection de mouvement (141) d'un sujet (140) par utilisation d'au moins deux dispositifs (120, 130) conçus pour envoyer et recevoir des signaux de radiofréquence (121, 131), dans lequel le système (110) comprend :
une unité de commande (111) pour la commande des au moins deux dispositifs (120, 130) de telle sorte qu'au moins l'un parmi les au moins deux dispositifs (120, 130) est un dispositif d'envoi (120) envoyant un signal de radiofréquence (121) avec une fréquence de signal d'envoi et de telle sorte qu'au moins l'un parmi les au moins deux dispositifs (120, 130) est un dispositif de réception (130) recevant un signal de radiofréquence (131) qui est indicatif de réflexions du signal de radiofréquence envoyé du sujet (140),
une unité de fourniture de fréquence de signal (112) pour la fourniture de la fréquence de signal d'envoi avec laquelle le signal de radiofréquence (121) a été envoyé, et
une unité de détection (113) pour la détection de mouvement (141) du sujet (140) par réalisation d'un captage Doppler passif en fonction du signal de radiofréquence reçu (131) et de la fréquence de signal d'envoi fournie,
**caractérisé en ce que** l'unité de détection (113) est conçue pour déterminer une excitation dans une plage de fréquences dans un spectre du signal de radiofréquence reçu (131), dans lequel l'excitation est sensiblement constante sur un laps de temps prédéterminé, et pour réaliser le captage Doppler passif en fonction du signal de radiofréquence reçu (131) par filtrage de la plage de fréquences dans le spectre du signal de radiofréquence reçu (131).

2. Système de détection selon la revendication 1, dans lequel l'unité de commande (111) est en outre conçue pour commander les au moins deux dispositifs (120, 130) de telle sorte que chacun des au moins deux dispositifs (120, 130) agit comme un dispositif d'envoi (120) envoyant chacun un signal de radiofréquence (121) avec une fréquence de signal différente et pour commander les au moins deux dispositifs (120, 130) de telle sorte que chacun agit comme un dispositif de réception (130) pour recevoir un signal de radiofréquence (131) qui est indicatif de réflexions du signal de radiofréquence envoyé (121) du sujet (140) correspondant au signal de radiofréquence envoyé (121) de l'autre dispositif respectif, dans lequel l'unité de détection (113) est conçue pour réaliser le captage Doppler passif en fonction des signaux de radiofréquence reçus (131).

3. Système de détection selon l'une quelconque des revendications 1 et 2, dans lequel l'unité de commande (111) est conçue pour commander le dispositif d'envoi (120) pour détecter des signaux de radiofréquence résultant de réflexions du signal de radiofréquence envoyé (121) envoyé par lui-même, et dans lequel l'unité de détection (113) est conçue pour surveiller les signaux de radiofréquence détectés et pour détecter un mouvement (141) d'un sujet (140) en outre en fonction des signaux de radiofréquence surveillés.

4. Système selon l'une quelconque des revendications précédentes, dans lequel l'unité de détection (113) est conçue pour déterminer un canal I et un canal Q en fonction du signal de radiofréquence reçu (131) et pour réaliser le captage Doppler passif en fonction du canal I et du canal Q.

5. Système de détection selon l'une quelconque des revendications précédentes, dans lequel l'unité de commande (111) est en outre conçue pour commander le dispositif d'envoi (120) pour envoyer un signal de radiofréquence supplémentaire avec une fréquence de signal différente de la fréquence de signal d'envoi et pour commander le dispositif de réception (130) pour recevoir un signal de radiofréquence supplémentaire résultant de la réflexion du signal de radiofréquence supplémentaire provenant du sujet (140) et dans lequel l'unité de détection (113) est conçue pour réaliser le captage Doppler passif en outre en fonction du signal de radiofréquence reçu supplémentaire.

6. Système de détection selon la revendication 5, dans lequel l'unité de détection (113) est conçue pour réaliser le captage Doppler passif en outre en fonction du signal de radiofréquence reçu par comparaison du signal de radiofréquence reçu supplémentaire avec le signal de radiofréquence reçu (131).

7. Système selon la revendication 6, dans lequel la comparaison se réfère à une soustraction du signal de radiofréquence reçu supplémentaire du signal de radiofréquence reçu (131) dans le domaine fréquentiel, dans lequel l'unité de détection (113) est conçue pour détecter un mouvement (141) en fonction du signal résultant de la soustraction.

8. Système selon la revendication 6, dans lequel la comparaison se réfère à la réalisation d'une analyse Doppler sur les deux signaux indépendamment l'un de l'autre et à la comparaison des résultats de l'analyse Doppler par rapport à une cohérence.

9. Système selon l'une quelconque des revendications précédentes, dans lequel l'unité de détection (113) est en outre conçue pour réaliser un captage de radiofréquence en fonction de l'amplitude du signal reçu et dans lequel l'unité de détection (113) est conçue pour réaliser un captage Doppler passif alternativement ou en complément d'une détection de radiofréquence en fonction d'un résultat de captage déterminé pour au moins l'un parmi les au moins deux dispositifs (120, 130) et/ou en fonction d'un état de dispositif d'au moins l'un parmi les au moins deux dispositifs (120, 130).

10. Système selon l'une quelconque des revendications précédentes, dans lequel deux paires de dispositifs sont utilisées pour la détection de mouvement, dans lequel l'unité de commande (111) est conçue pour commander chaque paire de dispositifs de telle sorte que chaque paire de dispositifs comprend au moins un dispositif d'envoi et un dispositif de réception, dans lequel l'unité de commande (111) est en outre conçue pour commander les dispositifs d'envoi et de réception de telle sorte qu'un signal de radiofréquence d'une fréquence différente est utilisé par les deux paires de dispositifs pour le captage Doppler passif, dans lequel l'unité de détection (113) est conçue pour réaliser le captage Doppler passif pour chaque paire de dispositifs indépendamment et pour détecter en outre un mouvement en fonction d'une comparaison des résultats de détection résultants.

11. Système selon la revendication 10, dans lequel le mouvement détecté se réfère à de minuscules mouvements ou vibrations du sujet.

12. Système selon l'une quelconque des revendications précédentes, dans lequel un résultat de la détection de mouvement est utilisé pour la commande d'une fonctionnalité des dispositifs.

13. Procédé de détection pour la détection de mouvement d'un sujet par utilisation d'au moins deux dispositifs conçus pour envoyer et recevoir des signaux de radiofréquence, dans lequel le procédé (200) comprend :
la commande (210) des au moins deux dispositifs de telle sorte qu'au moins l'un parmi les au moins deux dispositifs est un dispositif d'envoi envoyant un signal de radiofréquence avec une fréquence de signal d'envoi et de telle sorte qu'au moins l'un parmi les au moins deux dispositifs est un dispositif de réception recevant un signal de radiofréquence qui est indicatif de réflexions du signal de radiofréquence envoyé du sujet,
la fourniture (220) de la fréquence de signal d'envoi avec laquelle le signal de radiofréquence a été envoyé, et
la détection (230) de mouvement du sujet par réalisation d'un captage Doppler passif en fonction du signal de radiofréquence reçu et de la fréquence de signal d'envoi fournie,
**caractérisé en ce que** le procédé comprend en outre la détermination d'une excitation dans une plage de fréquences dans un spectre du signal de radiofréquence reçu (131), dans lequel l'excitation est sensiblement constante sur un laps de temps prédéterminé, et pour réaliser le captage Doppler passif en fonction du signal de radiofréquence reçu (131) par filtrage de la plage de fréquences dans le spectre du signal de radiofréquence reçu (131).

14. Produit programme informatique pour la détection de mouvement, dans lequel le produit programme informatique comprend des moyens de code de programme amenant un système de détection selon la revendication 1 à exécuter un procédé de détection selon la revendication 13.
